# EUROPÄISCHE PATENTANMELDUNG

(11) **EP 4 563 561 A1**
(43) Veröffentlichungstag der Anmeldung: **04.06.2025**
(21) Anmeldenummer: 24213332.0
(22) Anmeldetag: 15.11.2024
(51) Int. Cl.: C07C 7/04, C07C 7/00, C07C 11/08, B01D 1/28, B01D 3/00, B01D 3/14

(54) **ENERGIEEFFIZIENTES VERFAHREN ZUR ABTRENNUNG VON 1-BUTEN AUS EINEM KOHLENWASSERSTOFFSTROM UNTER EINSATZ EINES WÄRMETRÄGERS**

(30) Priorität: 29.11.2023 EP 23213041
(71) Anmelder: Evonik Oxeno GmbH & Co. KG, 45772 Marl (DE)
(72) Erfinder: Paul, Niklas, 45770 Marl (DE); Rix, Armin Matthias, 45770 Marl (DE); Schröder, Moritz, 48153 Münster (DE); Six, Tanita Valèrie, 44309 Dortmund (DE); Steenweg, Tanja, 44139 Dortmund (DE); Mellaerts, Wim, 2940 Hoevenen (BE); Knossalla, Johannes, 48351 Everswinkel (DE); Peitz, Stephan, 45739 Oer-Erkenschwick (DE); Stochniol, Guido, 45721 Haltern am See (DE); Laiblin, Tobias, 2930 Brasschaat (BE)
(74) Vertreter: Evonik Patent Association

(57) **Zusammenfassung**

Gegenstand der Erfindung ist ein Verfahren zur Abtrennung von 1-Buten aus einem C4-Kohlenwasserstoffstrom, der zumindest 1-Buten, 2-Buten, n-Butan und Isobutan enthält, in einer Abtrenneinheit, die mindestens zwei Destillationskolonnen DK1 und DK2 umfasst, wobei die Kondensationswärme mittels eines Wärmeträgers W nutzbar gemacht wird, um Energiekosten einzusparen und CO₂-Emission zu verringern.

## Beschreibung

Die vorliegende Erfindung betrifft ein Verfahren zur Abtrennung von 1-Buten aus einem C4-Kohlenwasserstoffstrom, der zumindest 1-Buten, 2-Buten, n-Butan und Isobutan enthält, in einer Abtrenneinheit, die mindestens zwei Destillationskolonnen DK1 und DK2 umfasst, wobei die Kondensationswärme mittels eines Wärmeträgers nutzbar gemacht wird, um Energiekosten einzusparen und CO₂-Emission zu verringern.

1-Buten kann in großen Mengen aus technischen C4-Kohlenwasserstoffströmen, beispielsweise dem C4-Schnitt aus Steamcrackern oder FCC-Einheiten, gewonnen werden. Diese C4-Kohlenwasserstoffströme bestehen im Wesentlichen aus Butadien, den Monoolefinen Isobuten, 1-Buten und den beiden 2-Butenen (cis- und trans-2-Buten) sowie den gesättigten Kohlenwasserstoffen Isobutan und n-Butan. Wegen der geringen Siedepunktsunterschiede der Inhaltsstoffe, deren geringen Trennfaktoren und der Ausbildung von Azeotropen ist eine ausschließlich destillative Aufarbeitung von C4-Kohlenwasserstoffströmen schwierig und nicht wirtschaftlich.

Üblicherweise wird deshalb zunächst das Butadien mittels Extraktivdestillation abgetrennt oder selektiv zu Butenen hydriert. Zurück bleibt jeweils ein C4-Kohlenwasserstoffstrom (sogenanntes Raffinat 1), der neben den gesättigten Kohlenwasserstoffen n-Butan und Isobutan die Olefine Isobuten, 1-Buten und 2-Butene enthält, während das Butadien maximal in geringen Mengen vorhanden ist.

Da die Siedepunkte von 1-Buten und Isobuten eng beieinanderliegen, kann aus entsprechenden C4-Kohlenwasserstoffströmen in der Regel kein 1-Buten über einfache Destillation wirtschaftlich abgetrennt werden. Das Isobuten wird deshalb möglichst weitgehend entfernt, beispielsweise über eine MTBE- oder ETBE-Synthese. Durch die Entfernung des Isobutens entsteht ein C4-Kohlenwasserstoffstrom (sogenanntes Raffinat 2), der die linearen Butene (1- und 2-Butene) und die gesättigten Kohlenwasserstoffe Isobutan und n-Butan enthält.

Die Abtrennung von 1-Buten aus derartigen C4-Kohlenwasserstoffströmen ist möglich und wird in der chemischen Industrie eingesetzt. Die Abtrennung erfolgt dabei in einer Destillationseinheit, die mindestens zwei Destillationskolonnen umfasst. In der ersten Destillationskolonne werden Isobutan und 1-Buten am Kopf anfallen und zur zweiten Destillationskolonne geleitet. In der zweiten Destillationskolonne werden dann Isobutan und 1-Buten voneinander getrennt. Ein solches Verfahren wird beispielsweise in der DE 10 2005 062 700 A1 offenbart.

Die für die Auftrennung des C4-Kohlenwasserstoffstroms notwendige Energie wird bei den bekannten Verfahren üblicherweise über Heizdampf in den Sumpf der beiden Destillationskolonnen eingebracht. Zwar steht an chemischen Produktionsstandorten Heizdampf in aller Regel zur Verfügung. In den für die betreffenden Trennaufgaben benötigten Mengen, bedeutet der Einsatz von Heizdampf einen nicht zu unterschätzenden Kostenfaktor. Zudem ist die Rückführung des gebrauchten Heizdampfs logistisch nicht immer einfach, da der Dampf nur innerhalb gewissen Spezifikationen (Druck, Temperatur, o. ä.) zurückgefahren werden dann. Darüber hinaus wird bei der Erzeugung von Heizdampf eine große Menge an CO₂ erzeugt.

Die Aufgabe der vorliegenden Erfindung bestand deshalb darin ein Verfahren bereitzustellen, bei dem Energie und CO₂ im Vergleich zu den bekannten Verfahren eingespart werden können und das in bestehende Anlagen integriert werden kann.

Diese Aufgabe wird durch die in Anspruch 1 vorgeschlagene Ausführung des Verfahrens gelöst. Bevorzugte Ausführungsformen sind in den Unteransprüchen angegeben. Das erfindungsgemäße Verfahren ist ein Verfahren zur Abtrennung von 1-Buten aus einem Raffinat-2-Strom, der zumindest 1-Buten, 2-Buten, n-Butan und Isobutan enthält, in einer Abtrenneinheit, die mindestens zwei Destillationskolonnen DK1 und DK2 umfasst, wobei
die erste Destillationskolonne DK1 mindestens einen Sumpfverdampfer SV1 und die zweite Destillationskolonne DK2 mindestens einen Sumpfverdampfer SV2 aufweist;
der Sumpfverdampfer SV1 mit einem Strom gespeist wird, der am unteren Ende der DK1 entnommen wird und nach Durchlaufen des jeweiligen Sumpfverdampfers wieder zur DK1 geführt wird;
der Sumpfverdampfer SV2 mit einem Strom gespeist wird, der am unteren Ende der DK2 entnommen wird und nach Durchlaufen des jeweiligen Sumpfverdampfers wieder zur DK2 geführt wird; wobei das Verfahren die folgenden Schritte umfasst
   (a) der Raffinat-2-Strom zur ersten Destillationskolonne DK1 geleitet wird und in der DK1 in mindestens einen Brüdenstrom BS1, der zumindest 1-Buten und Isobutan umfasst und am Kopf der DK1 entnommen wird, und in mindestens einen Sumpfstrom, der zumindest 1-Buten und 2-Buten umfasst und am Sumpf der DK1 entnommen wird, aufgetrennt wird;
   (b) der Brüdenstrom in mindestens zwei Teilströme BS1a und BS1b aufgetrennt wird;
   (c) Energie von BS1a auf einen flüssigen oder gasförmigen Wärmeträger W übertragen wird, wodurch ein Wärmeträger W1 entsteht;
   (d) ein von BS1a verschiedener Teil BS1b des Brüdenstroms BS1 zum Sumpfverdampfer SV2 geführt wird und dort Energie von BS1b auf den Strom im Sumpfverdampfer SV2 übertragen wird;
   (e) die Ströme BS1a und BS1b zumindest teilweise zur zweiten Destillationskolonne DK2 geleitet werden und in der DK2 in mindestens einen Brüdenstrom BS2, der zumindest Isobutan umfasst und am Kopf der DK2 entnommen wird, und in mindestens einen Produktstrom, der zumindest 1-Buten umfasst und am Sumpf der DK2 entnommen wird, aufgetrennt wird;
   (f) Energie von BS2 auf einen flüssigen oder gasförmigen Wärmeträger W übertragen wird, wodurch ein Wärmeträger W2 entsteht;
   (g) mindestens ein Teil des Wärmeträgers W2 verdichtet wird, wodurch ein verdichteter Wärmeträger W2.1 entsteht, der einen höheren Druck aufweist als der Wärmeträger W2;
   (h) der Wärmeträger W1 mit dem Wärmeträger W2.1 vermischt wird, wodurch ein vermischter Wärmeträger W3 entsteht:
   (i) mindestens ein Teil des vermischten Wärmeträgers W3 verdichtet wird, wodurch ein verdichteter Wärmeträger W3.1 entsteht, der einen höheren Druck aufweist als der vermischte Wärmeträger W3;
   (j) Energie vom verdichteten Wärmeträger W3.1 auf den Strom im Sumpfverdampfer SV1 übertragen wird.

Ein Vorteil des erfindungsgemäßen Verfahrens ist die Wärmeintegration über mindestens einen Wärmeträger, von dem Energie in dem Sumpfverdampfer SV1 auf den dort vorhandenen Strom und so Energie in den Sumpf eingetragen übertragen wird. Die Energieübertragung im Sumpfverdampfer SV1 in der ersten Destillationskolonne DK1 hat zur Folge, dass weniger oder gar kein Heizdampf mehr zur Beheizung der Destillationskolonne DK1 eingesetzt werden muss. Da auch der Sumpfverdampfer SV2 mit einem Teil des Brüdens betrieben wird, kann eine (fast) vollständige Verstromung des energieintensiven Verfahrens erzielt werden, was wiederum den Einsatz von grünem Strom ermöglicht. Dadurch werden erhebliche Mengen an Energiekosten und CO₂ eingespart.

Der Ausgangsstrom, aus dem das 1-Buten abgetrennt werden soll, ist nach der vorliegenden Erfindung ein Raffinat-2-Strom, der zumindest 1-Buten, 2-Buten, n-Butan und Isobutan enthält. Entsprechende Ströme sind auf dem Markt verfügbar, beispielsweise als C4-Schnitt aus Steamcrackern oder FCC-Einheiten. Es wurde in der Einleitung bereits erwähnt, dass Raffinat 2 dadurch entsteht, dass mehrfach ungesättigte C4-Kohlenwasserstoffe, insbesondere Butadien, und Isobuten aus dem Strom entfernt werden. Eine vollständige Entfernung ist aus wirtschaftlichen und technischen Gründen in vielen Fällen nicht möglich. Die Mengen an mehrfach ungesättigten C4-Kohlenwasserstoffen, insbesondere Butadien, und Isobuten sollten jedoch möglichst gering sein.

Vorzugsweise enthält das eingesetzte Raffinat 2 weniger als 1000 ppm, vorzugsweise weniger als 500 ppm Isobuten. Sind höhere Mengen an Isobuten im Ausgangstrom vorhanden, könnte zwischen die beiden Destillationskolonnen DK1 und DK2 eine MTBE- oder ETBE-Synthese (Methyl-tert.-butyl-ether = MTBE / Ethyl-tert.-butyl-ether = ETBE) erfolgen, um das Isobuten mit Methanol (für MTBE) oder Ethanol (ETBE) umzusetzen und anschließend das MTBE oder ETBE abzutrennen. So kann die Konzentration an Isobuten vor der zweiten Destillationskolonne DK2 stark reduziert werden. Isobuten würde in der zweiten Destillationskolonne nämlich im Sumpf und damit im 1-Buten anfallen.

Weiterhin bevorzugt enthält das im Verfahren der vorliegenden Erfindung eingesetzte Raffinat 2 weniger als 4 Gew.-% mehrfach ungesättigte C4-Kohlenwasserstoffe. In einer besonders bevorzugten Ausführungsform sollte die die Konzentration der mehrfach ungesättigten C4-Kohlenwasserstoffe weniger als 500 ppm betragen. Sollten die Ströme höhere Mengen an Butadien enthalten, könnte vorher eine Selektivhydrierung durchgeführt werden, bei der das Butadien zu Butenen und/oder Butanen umgesetzt wird. Entsprechende Verfahren sind dem Fachmann beispielsweise aus der EP 3 680 224 A1 bekannt.

Der eingesetzte Raffinat-2-Strom kann darüber hinaus gewisse Mengen an Wasser enthalten, insbesondere in einer Menge von 150 bis 4000 ppm. Es ist bevorzugt, dass das Wasser durch das hier beschriebene Verfahren zumindest teilweise abgetrennt wird. Das Wasser wird sich dabei in jeder der beiden Destillationskolonnen DK1 und DK2 im jeweiligen Brüdenstrom BS1 und BS2 anreichern und nach Kondensation als zweite flüssige Phase anfallen, die über Euter in den Destillatbehältern der DK1 und/oder der DK2 abgetrennt werden können. Die Sumpfprodukte der DK1 und der DK2 zeichnen sich durch sehr niedrige Gehalte an Butadien und Wasser, bevorzugt je unter 100 ppm, besonders bevorzugt unter 5 ppm aus.

Das erfindungsgemäße Verfahren wird in einer Abtrenneinheit durchgeführt, die mindestens die beiden Destillationskolonnen DK1 und DK2 umfasst. DK1 ist die erste Destillationskolonne und weist mindestens einen Sumpfverdampfer SV1 auf. DK2 ist die zweite Destillationskolonne und weist mindestens einen Sumpfverdampfer SV2 auf. In einer bevorzugten Ausführungsform weist die Destillationskolonne nur den Sumpfverdampfer SV1 auf. Es ist zudem bevorzugt, dass die Destillationskolonne DK2 nur den einen Sumpfverdampfer SV2 aufweist. Die Drücke in den beiden Destillationskolonnen DK1 und DK2 sollten insbesondere so gewählt werden müssen, dass Wärme in den Sumpfverdampfern übertragen werden kann. Die Begriffe "erste Destillationskolonne", "Destillationskolonne DK1" und "DK1" sind im Rahmen der vorliegenden Erfindung als synonym zu verstehen. Ebenso sind die sie Begriffe "zweite Destillationskolonne", "Destillationskolonne DK2" und "DK2" im Rahmen der vorliegenden Erfindung als synonym zu verstehen.

Über den Sumpfverdampfer SV1 wird die für die Trennaufgabe notwendige Energie in die erste Destillationskolonne DK1 eingebracht. Der Sumpfverdampfer SV1 wird mit einem Strom gespeist, der am unteren Ende der DK1 entnommen und nach Durchlaufen des jeweiligen Sumpfverdampfers wieder zur DK1 geführt wird. Der Strom wird beim Durchlaufen des Sumpfverdampfers SV1 erhitzt.

Vergleichbares gilt für den Sumpfverdampfer SV2 der zweiten Destillationskolonne DK2, durch den für die Trennaufgabe notwendige Energie eingetragen wird. Der Sumpfverdampfer SV2 wird dazu mit einem Strom gespeist wird, der am unteren Ende der DK2 entnommen und nach Durchlaufen des jeweiligen Sumpfverdampfers wieder zur DK2 geführt wird. Der Strom wird beim Durchlaufen des Sumpfverdampfers SV2 erhitzt und verdampft dabei zumindest teilweise.

Als "Sumpfverdampfer" werden erfindungsgemäß Verdampfer bezeichnet, die den Sumpf der jeweiligen Destillationskolonne beheizen. Ein derartiger Sumpfverdampfer ist üblicherweise außerhalb der jeweiligen Destillationskolonne angeordnet. Da in Sumpfverdampfern Energie, insbesondere Wärme, von einem Strom auf einen anderen übertragen wird, sind sie Wärmeüberträger. Über einen Abzug aus dem Sumpf der Destillationskolonne wird der zu verdampfende Strom abgezogen und dem Sumpfverdampfer zugeführt. Über mindestens einen Zulauf wird der verdampfte Strom gegebenenfalls mit einem Restanteil Flüssigkeit wieder in die jeweilige Destillationskolonne im Bereich des Sumpfs zurückgeführt.

Geeignete Verdampfer, die als Sumpfverdampfer eingesetzt werden können, sind zum Beispiel Naturumlaufverdampfer, Zwangsumlaufverdampfer, Zwangsumlaufverdampfer mit Entspannung, Kesselverdampfer, Fallfilmverdampfer oder Dünnschichtverdampfer. Als Wärmeübertrager für den Verdampfer wird bei Naturumlaufverdampfern und Zwangsumlaufverdampfern üblicherweise ein Rohrbündel oder Plattenapparat eingesetzt. Neben den genannten kann aber auch jede beliebige andere, dem Fachmann bekannte Verdampferbauart, die sich zum Einsatz an einer Destillationskolonne eignet, eingesetzt werden.

Das Raffinat 2, das zur ersten Destillationskolonne DK1 geleitet wird, wird in der Destillationskolonne DK1 in mindestens zwei Ströme aufgetrennt, d. h. in mindestens einen Brüdenstrom BS1, der zumindest 1-Buten und Isobutan umfasst und am Kopf der DK1 entnommen wird, und in mindestens einen Sumpfstrom, der zumindest 1-Buten und 2-Buten umfasst und am Sumpf der DK1 entnommen wird. Dieser Sumpfstrom kann zu einer Oligomerisierung geführt werden. Der Brüdenstrom BS1 kann am Kopf der Destillationskolonne auch in Form von mehreren Teilströmen BS1n, wobei n eine ganze Zahl und gleich der Anzahl der Teilströme ist, entnommen werden. Gleiches gilt auch für den Sumpfstrom. Im Sumpf der ersten Destillationskolonne DK1 liegt vorzugsweise eine Temperatur im Bereich von 40 bis 110 °C, vorzugsweise 50 bis 100 °C vor.

Grundsätzlich kann der Raffinat-2-Strom über eine oder mehrere Zulaufstellen in die erste Destillationskolonne DK1 geleitet werden. Sind mehrere Zulaufstellen für den Raffinat-2-Strom vorhanden, werden demnach mehrere voneinander getrennte Ströme in die Destillationskolonne geleitet. In den Ausführungsformen der vorliegenden Erfindung, in denen das der Raffinat-2-Strom als zwei oder mehrere voneinander getrennte Ströme in die Destillationskolonne DK1 geleitet wird, ist es vorteilhaft, wenn die Zulaufstellen der einzelnen Ströme im Wesentlichen auf der gleichen Höhe an der Destillationskolonne DK1 liegen.

Im Folgenden werden der Druck und die Temperatur des Brüdenstroms BS1 angegeben. Dies bezieht sich insbesondere auf den Druck und die Temperatur des mindestens einen Brüdenstroms BS1, wenn er der Destillationskolonne DK1 entnommen wird. Der Druck des Brüdenstroms BS1 liegt insbesondere im Bereich von 6 bis 15 bar absolut, vorzugsweise im Bereich von 7,5 bis 13 bar absolut. Die Temperatur des Brüdenstroms BS1 liegt insbesondere im Bereich von 45 °C bis 120 °C, bevorzugt im Bereich von 48 °C bis 100 °C, weiterhin bevorzugt im Bereich von 50 °C bis 90 °C, weiterhin bevorzugt im Bereich von 55 °C bis 80 °C, besonders bevorzugt im Bereich von 60 °C bis 80 °C.

Als Destillationskolonne DK1 für die Auftrennung des Raffinat-2-Stroms kann jede beliebige, dem Fachmann bekannte Destillationskolonne eingesetzt werden. Bevorzugt enthält die Destillationskolonne DK1 Einbauten. Geeignete Einbauten sind zum Beispiel Böden, unstrukturierte Packungen (Füllkörper) oder strukturierte Packungen. Als Böden werden üblicherweise Glockenböden, Siebböden, Ventilböden mit festen oder beweglichen Ventilen, Tunnelböden oder Schlitzböden eingesetzt. Unstrukturierte Packungen sind im Allgemeinen Füllkörperschüttungen. Als Füllkörper werden üblicherweise Raschigringe, Pallringe, Berl-Sättel, SuperRinge/SuperRinge Plus oder Intalox^{®}-Sättel verwendet. Strukturierte Packungen werden zum Beispiel unter dem Handelsnamen Mellapak^{®} der Firma Sulzer vertrieben. Neben den genannten Einbauten sind dem Fachmann weitere geeignete Einbauten bekannt und können ebenfalls verwendet werden.

Bevorzugte Einbauten weisen einen geringen spezifischen Druckverlust pro theoretischer Trennstufe auf. Strukturierte Packungen und Füllkörper haben zum Beispiel einen deutlich kleineren Druckverlust pro theoretischer Trennstufe als Böden. Dies hat den Vorteil, dass der Druckverlust in der Destillationskolonne DK1 möglichst gering bleibt und somit die mechanische Leistung des Verdichters und die Temperatur des zu verdampfenden Raffinat-2-Stroms gering bleibt.

In einer besonders bevorzugten Ausführungsform der vorliegenden Erfindung umfasst die Destillationskolonne DK1 eine Vielzahl von Böden, vorzugsweise zwischen 150 und 300 Böden, weiterhin bevorzugt zwischen 170 und 220 Böden.

Die Entnahme von mindestens einem Brüdenstroms BS1, der zumindest 1-Buten und Isobutan umfasst, am Kopf der Destillationskolonne DK1 bedeutet im Rahmen der vorliegenden Erfindung insbesondere, dass der mindestens eine Brüdenstrom BS1 als Kopfstrom oder als Seitenabzug oberhalb der Einbauten in der Destillationskolonne DK1 entnommen wird.

Die Entnahme des mindestens einen Sumpfstroms, der zumindest 1-Buten und 2-Buten umfasst, am Sumpf der Destillationskolonne DK1 bedeutet im Rahmen der vorliegenden Erfindung insbesondere, dass der mindestens eine Sumpfstrom direkt am Sumpf oder am unteren Boden der Destillationskolonne DK1 entnommen wird.

Die Destillationskolonne DK1 wird vorzugsweise mit Rücklauf betrieben. Rücklauf bedeutet, dass der am oberen Ende der Destillationskolonne DK1 entnommene Brüdenstrom BS1 mindestens teilweise wieder der Destillationskolonne DK1 zugeführt wird. In den Fällen, in denen ein solcher Rücklauf eingestellt wird, beträgt das Rücklaufverhältnis dabei bevorzugt 2 bis 30, bevorzugter 5 bis 20, besonders bevorzugt 8 bis 15.

Ein Rücklauf kann dadurch eingestellt werden, dass am Kopf der Destillationskolonne DK1 ein Kondensator angebracht wird. In dem Kondensator wird Brüdenstrom BS1 teilweise kondensiert und der Destillationskolonne DK1 wieder zugeführt. Der Brüdenstrom oder ein Teil davon kann auch erst nach Verdichtung und Entspannung als Rücklauf auf die Destillationskolonne gegeben werden. Unter einem Rücklaufverhältnis wird allgemein und im Sinne dieser Erfindung das Verhältnis aus dem Anteil des aus der Kolonne entnommenen Massenstroms (kg/h), der wieder auf die Kolonne in flüssiger Form zurückgeführt wird (Rücklauf) zu dem Anteil dieses Massenstroms (kg/h), der in flüssiger Form oder gasförmiger Form von der jeweiligen Kolonne abgeführt wird, verstanden.

Nach der Entnahme des Brüdenstroms BS1 wird der Brüdenstrom BS1 in Schritt (b) in mindestens zwei Teilströme BS1a und BS1b aufgetrennt. Die Auftrennung kann grundsätzlich auf bekannte Weise, beispielsweise durch einen Splitter (Regelung an einem Verdichter und/oder einem Stellventil) erfolgen. Denkbar wäre hier auch eine Regelung, über die die Massenströme von BS1a und BS1b in Abhängigkeit eines bestimmten Parameters eingestellt werden.

Anschließend wird in Schritt (c) Energie von dem Teilstrom BS1a auf einen flüssigen oder gasförmigen Wärmeträger W übertragen, wodurch ein Wärmeträger W1 entsteht. Die zu übertragene Energie ist in diesem Fall vorzugsweise Wärme und es entsteht ein erwärmter Wärmeträger W1. Durch die Übertragung von Energie auf den Wärmeträger W wird sich der Energiegehalt von BS1a verringern, der Strom sich also abkühlen und/oder kondensieren.

Als Wärmeträger W kann jedes dem Fachmann geläufige Arbeitsmedium genutzt werden. Der Wärmeträger W wird vorzugsweise aus der Gruppe bestehend aus Wasser; Alkoholen; Alkohol-Wasser-Mischungen; Salz-Wasser-Lösungen; Ammoniak; Mineralöle, wie zum Beispiel Dieselöle; Thermalölen, wie zum Beispiel Silikonöle; biologische Ölen, wie zum Beispiel Limonen; und aromatischen oder aliphatischen Kohlenwasserstoffen, wie zum Beispiel Dibenzyltoluol ausgewählt, weiterhin bevorzugt Wasser, Methanol, Ethanol, Propanol, n-Pentan, n-Butan, n-Hexan, n-Propan oder Ammoniak, besonders bevorzugt Wasser ist.

Wird der Wärmeträger W in Schritt (c) in flüssiger Phase, also ein flüssiger Wärmeträger W, eingesetzt und diesem in Schritt (c) Energie, bevorzugt Wärme, zugeführt, wird der Wärmeträger W zumindest teilweise verdampft und dadurch ein gasförmiger Wärmeträger W1 erhalten. Wird der Wärmeträger W in Schritt (c) in gasförmiger Phase, also ein gasförmiger Wärmeträger W eingesetzt und diesem in Schritt (c) Energie, bevorzugt Wärme, zugeführt, wird ebenfalls ein gasförmiger Wärmeträger W1 erhalten.

Mit der Formulierung "flüssiger Wärmeträger" ist im Rahmen der vorliegenden Erfindung gemeint, dass > 50 Gew.-%, weiterhin bevorzugt > 55 Gew.-%, weiterhin bevorzugt > 75 Gew.-%, weiterhin bevorzugt > 90 Gew.-% und besonders bevorzugt > 99 Gew.-% des in Schritt (c) eingesetzten Wärmeträgers im flüssigen Aggregatzustand vorliegen, jeweils bezogen auf das Gesamtgewicht des in Schritt (c) eingesetzten Wärmeträgers.

Mit der Formulierung "gasförmiger Wärmeträger" ist im Rahmen der vorliegenden Erfindung gemeint, dass > 30 Gew.-%, weiterhin bevorzugt > 50 Gew.-%, weiterhin bevorzugt > 75 Gew.-%, weiterhin bevorzugt > 90 Gew.-% und besonders bevorzugt > 99 Gew.-% des in Schritt (c) eingesetzten Wärmeträgers im gasförmigen Aggregatzustand vorliegen, jeweils bezogen auf das Gesamtgewicht des in Schritt (c) eingesetzten Wärmeträgers.

Die Übertragung von Energie in Schritt (c) kann nach dem Fachmann bekannten Verfahren oder mit dem Fachmann bekannten Wärmetauschern, beispielsweise den bereits erwähnten Verdampfern, durchgeführt werden. Der Wärmetauscher kann in diesem Fall auch der Kondensator zur Kondensation von BS1a sein. Das hat den Vorteil, dass kein zusätzlicher Kondensator eingebaut werden muss.

Nach der beschriebenen Energieübertragung in Schritt (c) weist der Wärmeträger W1 gegenüber dem Wärmeträger W vorzugsweise eine erhöhte Temperatur und/oder einen erhöhten Druck auf. In einer bevorzugten Ausführungsform der vorliegenden Erfindung weist W1 eine Temperatur im Bereich von 30 °C bis 80 °C auf. Der Druck von W1 liegt vorzugsweise im Bereich von 1 bar bis 20 bar, bevorzugt 3 bar bis 12 bar auf.

Es versteht sich von selbst, dass der Wärmeträger W1 dem Wärmeträger W entspricht und sich W und W1 nur in ihrem jeweiligen Druck und/oder ihrer Temperatur und gegebenenfalls, wenn W als Flüssigkeit eingesetzt wurde, durch den Aggregatzustand unterscheiden.

Im Schritt (d) des erfindungsgemäßen Verfahrens wird mindestens ein von BS1a verschiedener Teil BS1b des Brüdenstroms BS1 zum Sumpfverdampfer SV2 geführt wird und dort im Sumpfverdampfer SV2 Energie von BS1b auf den im Sumpfverdampfer vorhandenen Strom übertragen. Durch den Schritt (d) sinkt die Energie von BS1b, so dass BS1b vorzugsweise mindestens teilweise kondensiert.

Die Übertragung von Energie von BS1b auf den Strom im Sumpfverdampfer SV2, bevorzugt die Beheizung des Stroms im Sumpfverdampfer SV2 durch BS1b erfolgt bevorzugt direkt. Direkte Übertragung bedeutet, dass BS1b und der Strom in SV2 sich nicht direkt kontaktieren, aber dass Energie, insbesondere Wärme, von BS1b auf den Strom im SV2 übergeht, ohne dass ein zusätzliches Wärmeüberträgermedium vorhanden ist. Als Sumpfverdampfer SV2 können die dem Fachmann geläufigen Wärmeüberträger bzw. Wärmetauscher, insbesondere Verdampfer, wie sie vorher bereits erwähnt worden sind, eingesetzt werden.

Nachdem die Ströme BS1a und BS1b die Energieübertragung auf den Wärmeträger W respektive den Sumpfverdampfer SV2 durchlaufen und Energie auf die jeweiligen Ströme übertragen haben werden diese Ströme BS1a und BS1b in Schritt (e) zumindest teilweise zur zweiten Destillationskolonne DK2 geleitet, wo dann die Auftrennung zwischen Isobutan und 1-Buten erfolgt, um einen möglichst reinen 1-Buten-Strom zu erhalten. Die Auftrennung erfolgt in der DK2 in mindestens einen Brüdenstrom BS2, der zumindest Isobutan umfasst und am Kopf der DK2 entnommen wird, und in mindestens einen Produktstrom, der zumindest 1-Buten umfasst und am Sumpf der DK2 entnommen wird. Die beiden Ströme BS1a und BS1b können dabei unabhängig voneinander als unterschiedliche Zulaufströme oder gemeinsam zur zweiten Destillationskolonne DK2 geführt werden.

Bevor die Ströme zur zweiten Destillationskolonne mit Schritt (e) zur zweiten Destillationskolonne geführt werden, werden die Ströme BS1a und BS1b nach einer bevorzugten Ausführungsform zum einem Flashbehälter geführt und dort entspannt, wodurch eine flüssige Phase FP1 der Ströme BS1a und BS1b anfällt. Der Flashbehälter kann zusätzlich einen Kondensator umfassen, um einen Teil der anfallenden gasförmigen Phase zu kondensieren.

In einer bevorzugten Ausführungsform der vorliegenden Erfindung werden die Ströme BS1a und BS1b gemeinsam zur zweiten Destillationskolonne geführt. Dazu werden die beiden Ströme insbesondere auf den gleichen Druck und auf die gleiche Temperatur gebracht. Sollen die Ströme BS1a und BS1b also gemeinsam zur Destillationskolonne geführt werden, ist es bevorzugt, dass die Ströme BS1a und BS1b in einem Flashbehälter vereint werden und als gemeinsame flüssige Phase FP1 anfallen.

Zumindest ein Teil FP1a der flüssigen Phase FP1 wird anschließend gemäß Schritt (e) zur Destillationskolonne DK2 geleitet. Dazu wird in einer besonders bevorzugten Ausführungsform eine Pumpe eingesetzt. Hier können dem Fachmann bekannte Pumpen eingesetzt werden. Geeignete Pumpen sind beispielsweise Chemie-Normpumpen.

Es ist weiterhin bevorzugt, dass ein von FP1a verschiedener Teil FP1b der flüssigen Phase FP1 als Rücklauf zur ersten Destillationskolonne DK1 zurückgeführt wird. Besonders bevorzugt ist es, dass dabei Energie vom Strom FP1b auf den Raffinat-2-Strom übertragen wird, bevor der Raffinat-2-Strom in die erste Destillationskolonne DK1 eingeleitet wird. Hierdurch wird der Raffinat-2-Strom vorgewärmt. Dies ist energetisch vorteilhaft, weil weniger Energie für die Trennaufgabe über die Sumpfverdampfer eingebracht werden muss. Die Übertragung von Energie von FP1b auf den Raffinat-2-Strom, bevorzugt die Beheizung des Raffinat-2-Stroms durch FP1b erfolgt bevorzugt direkt, d. h. ohne Einsatz eines (weiteren) Wärmeträgers. Dafür können dem Fachmann geläufige Wärmeüberträger bzw. Verdampfer, wie vorher beschrieben, eingesetzt werden.

In der zweiten Destillationskolonne DK2 werden die Ströme, die beide jeweils zumindest Isobutan und 1-Buten umfassen, gemäß Schritt (e) in mindestens einen Brüdenstrom BS2, der zumindest Isobutan umfasst und am Kopf der DK2 entnommen wird, und in mindestens einen Produktstrom, der zumindest 1-Buten umfasst und am Sumpf der DK2 entnommen wird, aufgetrennt.

Als Destillationskolonne DK2 für die Auftrennung der beiden Ströme VB1 und BS1b bzw. FP1a kann jede beliebige, dem Fachmann bekannte Destillationskolonne eingesetzt werden. Bevorzugt enthält die Destillationskolonne DK2 Einbauten. Geeignete Einbauten sind zum Beispiel Böden, unstrukturierte Packungen (Füllkörper) oder strukturierte Packungen. Als Böden werden üblicherweise Glockenböden, Siebböden, Ventilböden mit festen oder beweglichen Ventilen, Tunnelböden oder Schlitzböden eingesetzt. Unstrukturierte Packungen sind im Allgemeinen Füllkörperschüttungen. Als Füllkörper werden üblicherweise Raschigringe, Pallringe, Berl-Sättel, SuperRinge/SuperRinge Plus oder Intalox^{®}-Sättel verwendet. Strukturierte Packungen werden zum Beispiel unter dem Handelsnamen Mellapak^{®} der Firma Sulzer vertrieben. Neben den genannten Einbauten sind dem Fachmann weitere geeignete Einbauten bekannt und können ebenfalls verwendet werden.

Bevorzugte Einbauten weisen einen geringen spezifischen Druckverlust pro theoretischer Trennstufe auf. Strukturierte Packungen und Füllkörper haben zum Beispiel einen deutlich kleineren Druckverlust pro theoretischer Trennstufe als Böden. Dies hat den Vorteil, dass der Druckverlust in der Destillationskolonne DK2 möglichst gering bleibt und somit die mechanische Leistung des Verdichters und die Temperatur der zu verdampfenden beiden Ströme BS1a und BS1b bzw. FP1a gering bleibt.

In einer besonders bevorzugten Ausführungsform der vorliegenden Erfindung umfasst die zweite Destillationskolonne DK2 eine Vielzahl von Böden, vorzugsweise zwischen 150 und 300 Böden, weiterhin bevorzugt zwischen 170 und 220 Böden.

Die Entnahme mindestens einen Brüdenstroms BS2, der zumindest Isobutan umfasst, am Kopf der Destillationskolonne DK2 bedeutet im Rahmen der vorliegenden Erfindung insbesondere, dass der mindestens eine Brüdenstroms BS2 als Kopfstrom oder als Seitenabzug oberhalb der Einbauten in der Destillationskolonne DK2 entnommen wird.

Die Entnahme des mindestens einen Produktstroms, der zumindest 1-Buten umfasst, am Sumpf der Destillationskolonne DK2 bedeutet im Rahmen der vorliegenden Erfindung insbesondere, dass der mindestens eine Produktstrom direkt am Sumpf oder am unteren Boden der Destillationskolonne DK2 entnommen wird. Der Produktstrom enthält vorzugsweise mindestens 99 Gew.-% 1-Buten, weiterhin bevorzugt mindestens 99,5 Gew.-% 1-Buten, besonders bevorzugt mindestens 99,6 Gew.-% 1-Buten. Das 1-Buten ist das Zielprodukt des vorliegenden Verfahrens, weshalb der Produktstrom aus dem Verfahren ausgeschleust wird. Das 1-Buten kann beispielsweise als Co-Monomer bei der Herstellung von Polyethylen eingesetzt werden.

Hierbei gilt zu beachten, dass die Trennschärfe in der ersten Destillationskolonne DK1 letztlich die Reinheit vom 1-Buten im Produktstrom, der aus der zweiten Destillationskolonne DK2 entnommen wird, bestimmt. Butan wird nämlich in der DK2 ebenfalls als Schwersieder und deshalb mit dem 1-Buten anfallen. Dadurch würde das 1-Buten verunreinigt. Es sollte also darauf geachtet werden, dass die Auftrennung des Raffinat-2-Stroms in der DK1 möglichst so gefahren wird, dass kaum Butan zur DK2 gelangt. Deshalb ist es bevorzugt, dass der zur DK2 geführte Strom (BS1a und BS1b bzw. FP1a) maximal zwischen 500 und 900 ppm Butane enthält, bezogen auf die Gesamtmenge des Stroms.

Im Sumpf der zweiten Destillationskolonne DK2 liegt während des erfindungsgemäßen Verfahrens vorzugsweise eine Temperatur im Bereich von 30 bis 100 °C, vorzugsweise 45 bis 80 °C vor. Weiterhin bevorzugt liegt am Kopf der zweiten Destillationskolonne DK2 ein Druck im Bereich von 3 bis 12 bar absolut, vorzugsweise 5 bis 10 bar absolut vor.

Die Destillationskolonne DK2 kann weiterhin mit Rücklauf betrieben werden. Rücklauf bedeutet, dass der am oberen Ende der Destillationskolonne DK2 entnommene Brüdenstrom BS2 mindestens teilweise wieder der Destillationskolonne DK2 zugeführt wird. In den Fällen, in denen ein solcher Rücklauf eingestellt wird, beträgt das Rücklaufverhältnis dabei bevorzugt 10 bis 80, besonders bevorzugt 30 bis 50.

Ein Rücklauf kann dadurch eingestellt werden, dass am Kopf der Destillationskolonne DK2 ein Kondensator angebracht wird. In dem Kondensator wird der Brüdenstrom BS2 teilweise kondensiert und der Destillationskolonne DK2 wieder zugeführt. Unter einem Rücklaufverhältnis wird allgemein und im Sinne dieser Erfindung das Verhältnis aus dem Anteil des aus der Kolonne entnommenen Massenstroms (kg/h), der wieder auf die Kolonne in flüssiger Form zurückgeführt wird (Rücklauf) zu dem Anteil dieses Massenstroms (kg/h), der in flüssiger Form oder gasförmiger Form von der jeweiligen Kolonne abgeführt wird, verstanden.

Der an der Destillationskolonne DK2 anfallende Brüdenstrom BS2 wird zur Wärmeintegration eingesetzt. In Schritt (f) wird Energie vom Brüdenstrom BS2 auf einen flüssigen oder gasförmigen Wärmeträger W übertragen, wodurch ein Wärmeträger W2 entsteht. Die zu übertragene Energie ist in diesem Fall vorzugsweise Wärme und es entsteht ein erwärmter Wärmeträger W2. Durch die Übertragung von Energie auf den Wärmeträger W wird sich der Energiegehalt von BS2 verringern, der Strom sich also abkühlen und/oder kondensieren.

Als Wärmeträger W kann jedes dem Fachmann geläufige Arbeitsmedium genutzt werden. Der Wärmeträger W wird vorzugsweise aus der Gruppe bestehend aus Wasser; Alkoholen; Alkohol-Wasser-Mischungen; Salz-Wasser-Lösungen; Ammoniak; Mineralöle, wie zum Beispiel Dieselöle; Thermalölen, wie zum Beispiel Silikonöle; biologische Ölen, wie zum Beispiel Limonen; und aromatischen oder aliphatischen Kohlenwasserstoffen, wie zum Beispiel Dibenzyltoluol ausgewählt wird, weiterhin bevorzugt Wasser, Methanol, Ethanol, Propanol, n-Pentan, n-Butan, n-Hexan, n-Propan oder Ammoniak, besonders bevorzugt Wasser ist. Der Wärmeträger W, der in den Schritten (c) und (f) eingesetzt wird, ist vorzugsweise identisch. Besonders bevorzugt ist es, dass es einen Wärmeträgerkreislauf gibt, bei dem der Wärmeträger W die Schritte (c) und (f) bis (j) durchläuft.

Wird der Wärmeträger W in Schritt (f) in flüssiger Phase, also ein flüssiger Wärmeträger W, eingesetzt und diesem in Schritt (f) Energie, bevorzugt Wärme, zugeführt, wird der Wärmeträger W zumindest teilweise verdampft und dadurch ein gasförmiger Wärmeträger W2 erhalten. Wird der Wärmeträger W in Schritt (f) in gasförmiger Phase, also ein gasförmiger Wärmeträger W, eingesetzt und diesem in Schritt (f) Energie, bevorzugt Wärme, zugeführt, wird ebenfalls ein gasförmiger Wärmeträger W2 erhalten.

Mit der Formulierung "flüssiger Wärmeträger" ist im Rahmen der vorliegenden Erfindung gemeint, dass > 50 Gew.-%, weiterhin bevorzugt > 55 Gew.-%, weiterhin bevorzugt > 75 Gew.-%, weiterhin bevorzugt > 90 Gew.-% und besonders bevorzugt > 99 Gew.-% des in Schritt (f) eingesetzten Wärmeträgers im flüssigen Aggregatzustand vorliegen, jeweils bezogen auf das Gesamtgewicht des in Schritt (f) eingesetzten Wärmeträgers.

Mit der Formulierung "gasförmiger Wärmeträger" ist im Rahmen der vorliegenden Erfindung gemeint, dass > 30 Gew.-%, weiterhin bevorzugt > 50 Gew.-%, weiterhin bevorzugt > 75 Gew.-%, weiterhin bevorzugt > 90 Gew.-% und besonders bevorzugt > 99 Gew.-% des in Schritt (f) eingesetzten Wärmeträgers im gasförmigen Aggregatzustand vorliegen, jeweils bezogen auf das Gesamtgewicht des in Schritt (f) eingesetzten Wärmeträgers.

Die Übertragung von Energie in Schritt (f) kann nach dem Fachmann bekannten Verfahren oder mit dem Fachmann bekannten Wärmetauschern, beispielsweise den bereits erwähnten Verdampfern, durchgeführt werden. Der Wärmetauscher kann in diesem Fall auch der Kondensator zur Kondensation von BS2 sein. Dies hat den Vorteil, dass kein zusätzlicher Kondensator eingebaut werden muss.

Nach der beschriebenen Energieübertragung in Schritt (f) weist der Wärmeträger W2 gegenüber dem Wärmeträger W vorzugsweise eine erhöhte Temperatur und/oder einen erhöhten Druck auf. In einer bevorzugten Ausführungsform der vorliegenden Erfindung weist W1 eine Temperatur im Bereich von 30 °C bis 80 °C auf. Der Druck von W2 liegt vorzugsweise im Bereich von 1 bar bis 20 bar, bevorzugt 2 bar bis 8 bar auf. In einer bevorzugten Ausführungsform der vorliegenden Erfindung ist der Druck und/oder die Temperatur des Wärmeträgen W2 kleiner als der Druck und/oder die Temperatur des Wärmeträgen W1.

Nachdem in Schritt (f) Energie vom Brüdenstrom BS2 auf den Wärmeträger übertragen worden ist, kann BS2 als Isobutan-Strom IB1 aus dem Verfahren ausgeschleust werden. Bevor BS2 aber als IB1 aus dem Verfahren abgeführt wird, wird der Strom VB2 nach einer bevorzugten Ausführungsform der vorliegenden Erfindung zum einem Flashbehälter geführt und dort entspannt, wodurch eine flüssige Phase FP2 anfällt. Der Flashbehälter kann zusätzlich einen Kondensator umfassen, um einen Teil der anfallenden gasförmigen Phase zu kondensieren.

Zumindest ein Teil FP2a der flüssigen Phase FP2 kann anschließend als Isobutan-Strom IB1 aus dem Verfahren ausgeschleust werden. Dazu wird in einer besonders bevorzugten Ausführungsform eine Pumpe eingesetzt. Unter Umständen und bei ausreichendem Druckverhältnis wäre es auch möglich, dass keine Pumpe eingesetzt wird. Sofern eine Pumpe eingesetzt wird, können dem Fachmann bekannte Pumpen eingesetzt werden. Geeignete Pumpen sind beispielsweise Chemie-Normpumpen. Es ist weiterhin bevorzugt, dass ein von FP2a verschiedener Teil FP2b der flüssigen Phase FP1 als Rücklauf zur ersten Destillationskolonne DK2 zurückgeführt wird.

Im anschließenden Schritt (g) wird mindestens ein Teil des Wärmeträgers W2 verdichtet, wodurch ein gegenüber W2 verdichteter Wärmeträger W2.1 entsteht, der einen höheren Druck aufweist als der Wärmeträger W2. Der Druck von W2.1 liegt nach dem Verdichten vorzugsweise im Bereich von 1 bar bis 20 bar, bevorzugt 3 bar bis 12 bar auf. Weiterhin bevorzugt sind der Druck des verdichteten Wärmeträgers W2.1 und des Wärmeträgers W1 identisch oder weichen maximal um ± 10% voneinander ab.

Das Verdichten des mindestens einen Teils des Wärmeträgers W2 in Schritt (g) kann auf jede beliebige, dem Fachmann bekannte Art erfolgen. So kann die Verdichtung zum Beispiel mechanisch und einstufig oder mehrstufig durchgeführt werden. Einstufig bedeutet in diesem Zusammenhang, dass eine Verdichtung von einem auf ein anderes Druckniveau stattfindet. Mehrstufig bedeutet, dass erst auf ein Druckniveau X und dann von X auf das Druckniveau Y verdichtet wird. Bei einer mehrstufigen Verdichtung können mehrere Verdichter gleicher Bauart oder Verdichter unterschiedlicher Bauart eingesetzt werden. Eine mehrstufige Verdichtung kann mit einer oder mehreren Verdichtermaschinen erfolgen. Der Einsatz einer einstufigen Verdichtung oder einer mehrstufigen Verdichtung ist vom Verdichtungsverhältnis und somit davon abhängig, auf welchen Druck der Wärmeträger W2 verdichtet werden soll.

Der Wärmeträger W1 in Schritt c) weist vorzugsweise einen höheren Druck auf als der Wärmeträger W2 in Schritt f). Durch die Verdichtung in Schritt g) wird der Wärmeträger W2 im Vergleich mit W1 auf den gleichen oder einen ähnlichen Druck gebracht. Die Formulierung "ähnlicher Druck" bedeutet in diesem Zusammenhang, dass sich der Druck von W1 und der Druck des verdichteten Wärmeträgers W2.1 nach der Verdichtung um weniger als 5% voneinander unterscheiden, vorzugsweise um weniger als 1% voneinander unterscheiden.

Als Verdichter im erfindungsgemäßen Verfahren, insbesondere zum Verdichten des Wärmeträgers W2, eignet sich jeder beliebige, dem Fachmann bekannte Verdichter, bevorzugt mechanische Verdichter, mit dem sich Gasströme verdichten lassen. Geeignete Verdichter sind zum Beispiel ein- oder mehrstufige Getriebeturboverdichter, Kolbenverdichter, Schraubenverdichter, Zentrifugalverdichter oder Axialverdichter.

Der verdichtete Wärmeträger W2.1 wird im nachfolgenden Schritt (h) mit dem Wärmeträger W1 vermischt, wodurch ein vermischter Wärmeträger W3 entsteht. Die Vermischung kann ohne besondere Einbauten einfach durch das Zusammenführen der beiden Rohrleitungen erfolgen. Dies ist dem Fachmann grundsätzlich geläufig. Die Vermischung der beiden Wärmeträger W2.1 und W1 hat den Vorteil, dass die Überhitzung des Wärmeträgers W1 zur Überhitzung des Wärmeträgers W2.1 beitragen kann. Dadurch muss kein weiterer Wärmetauscher vorhanden sein, bevor der vermischte Wärmeträger W3 im anschließenden Schritt (i) verdichtet wird.

Im bereits erwähnten Schritt (i) wird mindestens ein Teil des Wärmeträgers W3 verdichtet, wodurch ein gegenüber W3 verdichteter Wärmeträger W3.1 entsteht, der einen höheren Druck aufweist als der Wärmeträger W3. Der Druck von W3.1 liegt nach dem Verdichten vorzugsweise im Bereich von 5 bar bis 30 bar, bevorzugt 10 bar bis 20 bar auf.

Das Verdichten des mindestens einen Teils des Wärmeträgers W3 in Schritt (i) kann auf jede beliebige, dem Fachmann bekannte Art erfolgen. So kann die Verdichtung zum Beispiel mechanisch und einstufig oder mehrstufig durchgeführt werden. Einstufig bedeutet in diesem Zusammenhang, dass eine Verdichtung von einem auf ein anderes Druckniveau stattfindet. Mehrstufig bedeutet, dass erst auf ein Druckniveau X und dann von X auf das Druckniveau Y verdichtet wird. Bei einer mehrstufigen Verdichtung können mehrere Verdichter gleicher Bauart oder Verdichter unterschiedlicher Bauart eingesetzt werden. Eine mehrstufige Verdichtung kann mit einer oder mehreren Verdichtermaschinen erfolgen. Der Einsatz einer einstufigen Verdichtung oder einer mehrstufigen Verdichtung ist vom Verdichtungsverhältnis und somit davon abhängig, auf welchen Druck der Wärmeträger W3 verdichtet werden soll.

Als Verdichter im erfindungsgemäßen Verfahren, insbesondere zum Verdichten des Wärmeträgers W3, eignet sich jeder beliebige, dem Fachmann bekannte Verdichter, bevorzugt mechanische Verdichter, mit dem sich Gasströme verdichten lassen. Geeignete Verdichter sind zum Beispiel ein- oder mehrstufige Getriebeturboverdichter, Kolbenverdichter, Schraubenverdichter, Zentrifugalverdichter oder Axialverdichter.

Im Schritt (j) des erfindungsgemäßen Verfahrens wird Energie vom verdichteten Wärmeträger W3.1 auf den Strom im Sumpfverdampfer SV1 übertragen. Durch den Schritt (j) sinkt die Energie von W3.1, so dass W3.1 vorzugsweise mindestens teilweise kondensiert. Die Phrase Übertragung von Energie bedeutet erfindungsgemäß insbesondere Beheizung, also Übertragung von Energie in Form von Wärme.

Die Übertragung von Energie von W3.1 auf den Strom im Sumpfverdampfer SV1, bevorzugt die Beheizung des Stroms im Sumpfverdampfer SV1 durch W3.1 erfolgt bevorzugt direkt. Direkte Übertragung bedeutet, dass W3.1 und der Strom in SV1 sich zwar nicht direkt kontaktieren, aber dass Energie, insbesondere Wärme, von W3.1 auf den Strom im SV1 übergeht, ohne dass ein zusätzliches Wärmeüberträgermedium vorhanden ist. Als Sumpfverdampfer SV1 können die dem Fachmann geläufigen Wärmeüberträger bzw. Wärmetauscher, insbesondere Verdampfer, eingesetzt werden.

Die Übertragung von Energie in Schritt (j) kann nach dem Fachmann bekannten Verfahren oder mit dem Fachmann bekannten Wärmetauschern durchgeführt werden. Geeignete Verdampfer, die als Wärmetauscher eingesetzt werden können, sind zum Beispiel Naturumlaufverdampfer, Zwangsumlaufverdampfer, Zwangsumlaufverdampfer mit Entspannung, Kesselverdampfer, Fallfilmverdampfer oder Dünnschichtverdampfer. Neben den genannten kann aber auch jede beliebige andere, dem Fachmann bekannte Verdampferbauart, die sich zum Einsatz an einer Destillationskolonne eignet, eingesetzt werden.

In der grundsätzlichen Ausgestaltung der vorliegenden Erfindung werden die beiden Ströme W2 und W3 jeweils mit einem einzigen Verdichter verdichtet. In einer bevorzugten Ausführungsform der vorliegenden Erfindung werden die beiden Ströme W2 und W3 in einem einzigen, vorzugsweise mehrstufigen Verdichter verdichtet. Die Anzahl der notwendigen Stufen hängt davon ab, welches Verdichtungsverhältnis angestrebt wird.

Der Wärmeträger wird, wie erwähnt, vorzugsweise im Kreis gefahren. Damit ist gemeint, dass der Wärmeträger W3.1 nach der Energieübertragung in Schritt (j) zurückgefahren und wieder in den Schritten (c) und (f) eingesetzt wird. Dafür wird nach einer bevorzugten Ausführungsform der vorliegenden Erfindung im gesamten Verfahren nur ein einziger Wärmeträger eingesetzt wird. Der in Schritt (c) und in Schritt (f) eingesetzte Wärmeträger W ist also identisch. Der Wärmeträger W3.1 kann außerdem abgekühlt werden, bevor er als Wärmeträger W teilweise zu Schritt (c) und teilweise zu Schritt (f) geleitet wird. Grund dafür ist, dass der Wärmeträger W, der die Wärmeenergie aus den Kondensationen der Brüdenströme BS1 und BS2 aufnimmt, sollte vorzugsweise auf einem bestimmen Druck- und/oder Temperaturnniveau sein, um die gewünschte Menge an Wärmeenergie in den Wärmetauschern aufnehmen zu können. Es kann dafür notwendig sein, dass der Wärmeträger W3.1 nach der Energieübertragung im Sumpfverdampfer gekühlt werden muss. Um diese Energie zu nutzen kann die Kühlung in einem Wärmetauscher erfolgen, durch den der Wärmeträger W2 vorgewärmt wird. Dias wiederum kann dazu führen, dass weniger Energie durch den bzw. die Verdichter eingebracht werden muss.

Nach der Energieübertragung in Schritt (j) des erfindungsgemäßen Verfahrens kann der Wärmeträger W3.1 zur Vorwärmung des Raffinat-2-Stroms verwendet werden, bevor der Raffinat-2-Strom in die erste Destillationskolonne geleitet wird. Anschließend würde der Wärmeträger W3.1 nach der Vorwärmung zurückgeführt und als Wärmeträger W teilweise zu Schritt (c) und teilweise zu Schritt (f) geleitet werden. Ist keine Vorwärmung vorhanden, wird der Wärmeträger W3.1 ohne diesen zusätzlichen Schritt zurückgeführt und als Wärmeträger W teilweise zu Schritt (c) und teilweise zu Schritt (f) geleitet werden.

Die Wärmeintegration durch die hier beschriebene Wärmepumpe ließe sich auch mit anderen Maßnahmen zur Wärmeintegration kombinieren. Möglich wäre es hier noch eine oder mehrere Brüdenverdichtungen vorzusehen.

Die vorliegende Erfindung wird nachfolgend anhand von Abbildungen erläutert. Die Abbildungen dienen der Veranschaulichung, sind aber nicht einschränkend zu verstehen.

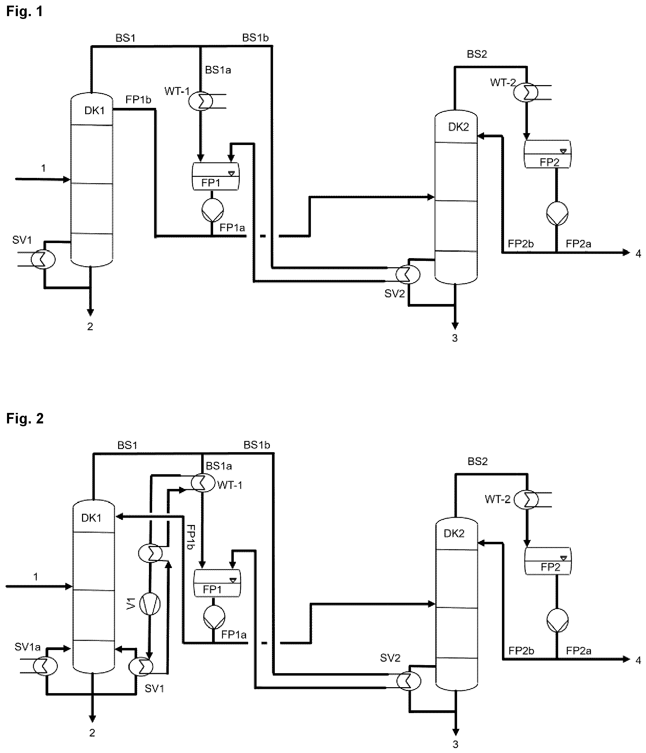

Fig. 1 zeigt eine Ausgestaltung nach dem Stand der Technik, eine klassiche Zweidruckschaltung (s. Beispiel 1). Der Raffinat-2-Strom (1) wird zur ersten Destillationskolonne DK1 geführt und dort in einen Brüdenstrom BS1, der zumindest 1-Buten und Isobutan umfasst und am Kopf der DK1 entnommen wird, und in einen Sumpfstrom (2), der zumindest 1-Buten und 2-Buten umfasst und am Sumpf der DK1 entnommen wird. Dieser Strom (2) kann zu einer Oligomerisierung (nicht eingezeichnet) geführt werden. Die Beheizung des Sumpfes der Kolonne DK1 im Sumpfverdampfer SV1 wird beispielsweise mittels Heizdampf erfolgen. Der Brüdenstrom BS1 wird in die beiden Brüdenströme BS1a und BS1b aufgetrennt. Der Teil BS1b des Brüdenstroms wird ohne zusätzliche Verdichtung zum Sumpfverdampfer SV2 geführt und überträgt dort Energie auf den dort vorhandenen Strom, der aus dem Sumpf kommend erhitzt und dann wieder zurückgeführt wird. BS1a wird mithilfe eines Wärmetauschers WT-1 heruntergekühlt. Die beiden Ströme BS1a und BS1b werden zu einem Flashbehälter gefahren werden, wo durch die Entspannung eine flüssige Phase FP1 anfällt. Diese flüssige Phase wird in die beiden Ströme FP1a, der zur zweiten Destillationskolonne DK2 geführt wird, und FP1b, der als Rücklauf zur ersten Destillationskolonne geleitet wird, aufgetrennt. In der Destillationskolonne 2 werden die Ströme in einen Brüdenstrom BS2, der zumindest Isobutan umfasst und am Kopf der DK2 entnommen wird, und in einen Produktstrom (3), der zumindest 1-Buten umfasst und am Sumpf der DK2 entnommen wird, aufgetrennt. Der Brüdenstrom BS2 wird in einem Wärmetauscher WT-2 gekühlt und zu einem Flashbehälter gefahren werden, wo durch die Entspannung eine flüssige Phase FP2 anfällt. Von der Phase FP2, wird ein Teil FP2a als Isobutan-Strom (4) das Verfahren verlassen und ein anderer Teil FP2b als Rücklauf zur zweiten Destillationskolonne zurückgeführt.
Fig. 2 zeigt eine nicht erfindungsgemäße Ausführungsform, die in weiten Teilen mit der in Fig. 1 dargestellten Ausführungsform identisch ist. Der Unterschied besteht darin, dass im Wärmetauscher WT-1 Energie vom Strom BS1a auf einen Wärmeträger übertragen wird, der Wärmeträger mithilfe eines Verdichters V1 verdichtet und anschließend zur Beheizung des Sumpfs der DK1 im Sumpfverdampfer SV1 eingesetzt wird. Es ist möglich, dass ein zusätzlicher Sumpfverdampfer SV1a notwendig wird, um den Sumpf der DK1 ausreichend beheizen zu können.

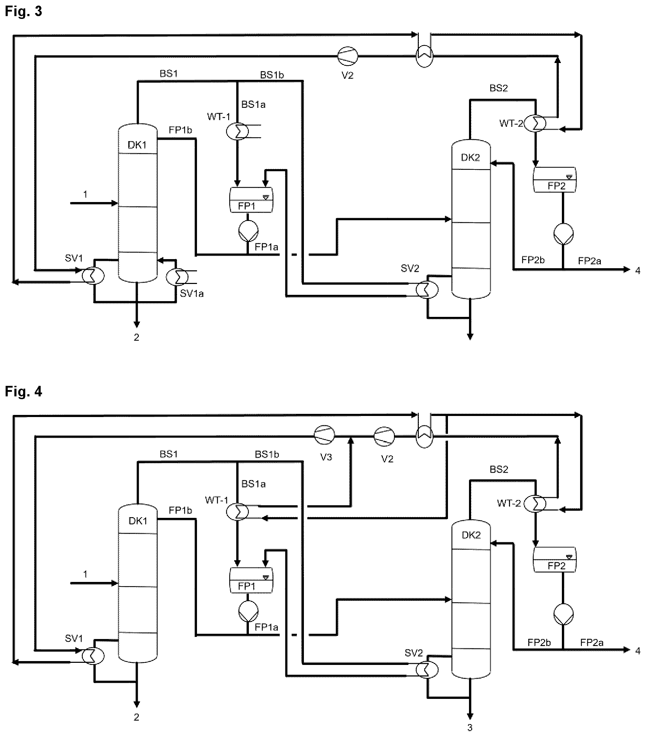

Fig. 3 zeigt eine nicht erfindungsgemäße Ausführungsform, die in weiten Teilen mit den in Fig. 1 dargestellten Ausführungsform identisch ist. Der Unterschied besteht darin, dass im Wärmetauscher WT-2 Energie vom Strom BS2 auf einen Wärmeträger übertragen wird, der Wärmeträger mithilfe eines Verdichters V2 verdichtet und anschließend zur Beheizung des Sumpfs der DK1 im Sumpfverdampfer SV1 eingesetzt wird. Bei der Rückführung kann der der zurückgeführte Wärmeträger im Wärmetauscher WT-3 zur Vorwärmung des Wärmeträgers vor der Verdichtung eingesetzt werden. Es ist auch hier möglich, dass ein zusätzlicher Sumpfverdampfer SV1a notwendig wird, um den Sumpf der DK1 ausreichend beheizen zu können.
Fig. 4 zeigt eine erfindungsgemäße Ausführungsform, die in weiten Teilen mit den in Fig. 1 bis Fig. 3 dargestellten Ausführungsformen identisch ist. Der Unterschied besteht darin, dass die beiden Wärmepumpen aus Fig. 2 und Fig.3 kombiniert werden. Im Wärmetauscher WT-2 wird dabei Energie vom Strom BS2 auf einen Wärmeträger übertragen und der Wärmeträger im Verdichter V2 verdichtet. Im Wärmetauscher WT-1 wird Energie vom Strom BS1a auf einen Wärmeträger übertragen und die beiden Wärmeträger vermischt. Der vermischte Wärmeträger wird dann in einem Verdichter V3 verdichtet und zur Beheizung des Sumpfs der DK1 im Sumpfverdampfer SV1 eingesetzt wird. Bei dieser Ausführungsform wird kein weiterer Sumpfverdampfer an der DK1 benötigt.

### Beispiele

Für alle nachfolgenden Beispiele wurde ein Raffinat 2-Strom von 55t/h eingesetzt. Der Raffinat-2-Stroms hat die folgende Zusammensetzung: 1-Buten 45,1 % / n-Butan 22,4% / trans-2-Buten 15,9% / cis-2-Buten 8,9% / Isobutan 7,4% / Isobuten 45 ppm und Wasser 590 ppm.

Die für den Betrieb, der in den Beispielen dargestellten Anlagen zur Abtrennung von 1 -Buten aus Raffinat 2 notwendige Menge an Energie wurde anhand einer Simulation mittels Aspen V10 errechnet. Die Stoffdaten wurden durch Betriebsdaten und Betriebsversuche validiert.

### Beispiele

### Beispiel 1 (nicht erfindungsgemäß):

In der Ausführungsform nach Fig. 1 wird ausschließlich eine direkte Wärmeintegration zwischen der Kolonne DK1 und DK2 durchgeführt (wie in DE 10 2005 062 700 A1 offenbart). Hierbei wird die Kolonne DK1 bei einem Kopfdruck von 11 bar und die Kolonne DK2 von 7 bar betrieben. So kann mit einem Teil des Brüdenstroms BS1 über den Sumpfverdampfer SV2 an der DK2 Wärme übertragen werden. Die Wärmemenge, die für eine 1-Butenmenge von 10,3 t/h bei einer Reinheit von 99,6% 1-Buten erforderlich ist, beträgt 10,3 MW in der DK2. Hierzu wird ein Brüdenstrom von 125,5 t/h der DK1 verwendet, um die DK2 zu beheizen. Nichtsdestotrotz müssen an der DK1 15,9 MW über eine externe Wärmequelle/Medium (z. B. Heizdampf) eingetragen werden.

### Beispiel 2 (nicht erfindungsgemäß):

Die erfindungsgemäße Verschaltung sieht vor die nicht genutzte Kondensationswärme an der DK1 nutzbar zu machen. Hier wird nur die Kondensationswärme der DK 1 von 4 MW über eine Wärmepumpe nutzbar gemacht. Die Kolonne DK1 wird wie im Beispiel 1 bei einem Kopfdruck von 11 bar abs. und einer Kopftemperatur von 72°C betrieben. Demzufolge könnte die Kondensationswärme der DK1 genutzt werden, um Methanol bei 1,1 bar zu verdampfen. Das gasförmige Methanol wird dann über einen Vorwärmer gefahren, um einen Tropfenschlag im nachfolgenden Verdichter zu vermeiden. Methanol wird auf ein Druck- bzw. Temperaturniveau gebracht, sodass die Wärme über den SV1 übertragen werden kann. Hierzu sind 450 kW elektrische Leistung erforderlich. Das kondensierte Methanol wird aus dem SV1 über die Vorwärmung wieder zum Kondensator der DK1 gefahren. Die externe Heizleistung kann so von 15,9 MW auf 11,45 MW reduziert werden. Die zusätzliche externe Heizleistung wird über den zusätzlichen Verdampfer SV1a eingetragen.

### Beispiel 3 (nicht erfindungsgemäß):

Die erfindungsgemäße Verschaltung sieht vor die nicht genutzte Kondensationswärme nutzbar zu machen. Hier wird nur die Kondensationswärme der DK2 von 9,9 MW über eine Wärmepumpe nutzbar gemacht. Die Kolonne DK2 wird wie im Beispiel 1 bei einem Kopfdruck von 7 bar abs. / Kopftemperatur von 50°C betrieben. Methanol - wie in Beispiel 2 - eignet sich hier nicht als Wärmeträger, denn Methanol müsste im Vakuum verdampft werden. In diesem Beispiel wird n-Pentan als Arbeitsmedium eingesetzt.

Die Kondensationswärme der DK2 wird genutzt, um n-Pentan bei 1,4 bar abs. zu verdampfen. Das gasförmige n-Pentan muss einer Überhitzung vor der anschließenden Verdichtung unterzogen werden. Um das n-Pentan für die DK1 nutzbar zu machen, wird es auf ein Druckniveau von 5 bar abs. verdichtet. Dies erfordert eine elektrische Leistung von 2000 kW. Durch die erfindungsgemäße Verschaltung können 11,9 MW der externen Heizleistung mit 2000 kW elektrischer Leistung subsituiert werden. Die noch fehlenden 4 MW an externer Heizleistung wird über den zusätzlichen Verdampfer SV1a eingetragen.

### Beispiel 4 (erfindungsgemäß):

Die erfindungsgemäße Verschaltung sieht vor die nicht genutzte Kondensationswärme nutzbar zu machen. Hier wird die Kondensationswärme beider Kolonnen DK1 und DK2 von insgesamt 13,5 MW über eine mehrstufige Wärmepumpe nutzbar gemacht. Die Kolonne DK2 wird wie im Beispiel 1 bei einem Kopfdruck von 7 bar abs. / Kopftemperatur von 50°C betrieben. Die Kondensationswärme der DK2 wird genutzt, um n-Pentan bei 1,4 bar abs. zu verdampfen. Das gasförmige n-Pentan muss einer Überhitzung vor der anschließenden Verdichtung unterzogen werden. Das gasförmige n-Pentan wird zunächst auf ein Druckniveau von 2,1 bar abs. mit einer ersten Verdichterstufe verdichtet. Ferner wird die Kondensationswärme der DK1 genutzt, um n-Pentan bei 2,1 bar abs. zu verdampfen. Der gasförmige n-Pentanstrom, der aus der Kondensationswärme der DK1 entsteht wird von einer zweiten Verdichterstufe zusammen mit dem bereits verdichten n-Pentanstrom eingesaugt und auf ein Druckniveau von 5 bar abs. verdichtet. Insgesamt werden 3,2 MW elektrische Leistung für die vollständige Elektrifizierung benötigt.

Nachfolgend sie die Ergebnisse der Beispiele 1 bis 4 in der Tabelle 1 zusammengefasst.

**Tabelle 1: Zusammenfassung Beispiele**

| | Beispiel 1 | Beispiel 2 | Beispiel 3 | Beispiel 4 |
|---|---|---|---|---|
| Externe Heizleistung [MW] | 15,9 | 11,45 | 4 | 0 |
| Elektrische Leistung Verdichter [MW] | 0 | 0,55 | 2 | 3,2 |

Es zeigt sich, dass die erfindungsgemäße Ausgestaltung des Verfahrens der 1-Butenabtrennung dazu führt, dass deutlich weniger externe Heizleistung eingesetzt werden muss als bei der bekannten Lösung. Das Einsparpotential ist also erheblich. Die dazu zusätzlich erforderliche elektrische Leistung zum Betrieb der Verdichter ist deutlich geringer und kann bei Einsatz von grünem Strom einen CO₂-neutralen Betrieb ermöglichen.

## Patentansprüche

1. Verfahren zur Abtrennung von 1-Buten aus einem Raffinat-2-Strom, der zumindest 1-Buten, 2-Buten, n-Butan und Isobutan enthält, in einer Abtrenneinheit, die mindestens zwei Destillationskolonnen DK1 und DK2 umfasst, wobei
die erste Destillationskolonne DK1 mindestens einen Sumpfverdampfer SV1 und die zweite Destillationskolonne DK2 mindestens einen Sumpfverdampfer SV2 aufweist;
der Sumpfverdampfer SV1 mit einem Strom gespeist wird, der am unteren Ende der DK1 entnommen wird und nach Durchlaufen des jeweiligen Sumpfverdampfers wieder zur DK1 geführt wird;
der Sumpfverdampfer SV2 mit einem Strom gespeist wird, der am unteren Ende der DK2 entnommen wird und nach Durchlaufen des jeweiligen Sumpfverdampfers wieder zur DK2 geführt wird; wobei das Verfahren die folgenden Schritte umfasst
(a) der Raffinat-2-Strom zur ersten Destillationskolonne DK1 geleitet wird und in der DK1 in mindestens einen Brüdenstrom BS1, der zumindest 1-Buten und Isobutan umfasst und am Kopf der DK1 entnommen wird, und in mindestens einen Sumpfstrom, der zumindest 1-Buten und 2-Buten umfasst und am Sumpf der DK1 entnommen wird, aufgetrennt wird;
(b) der Brüdenstrom in mindestens zwei Teilströme BS1a und BS1b aufgetrennt wird;
(c) Energie von BS1a auf einen flüssigen oder gasförmigen Wärmeträger W übertragen wird, wodurch ein Wärmeträger W1 entsteht;
(d) ein von BS1a verschiedener Teil BS1b des Brüdenstroms BS1 zum Sumpfverdampfer SV2 geführt wird und dort Energie von BS1b auf den Strom im Sumpfverdampfer SV2 übertragen wird;
(e) die Ströme BS1a und BS1b zumindest teilweise zur zweiten Destillationskolonne DK2 geleitet werden und in der DK2 in mindestens einen Brüdenstrom BS2, der zumindest Isobutan umfasst und am Kopf der DK2 entnommen wird, und in mindestens einen Produktstrom, der zumindest 1-Buten umfasst und am Sumpf der DK2 entnommen wird, aufgetrennt wird;
(f) Energie von BS2 auf einen flüssigen oder gasförmigen Wärmeträger W übertragen wird, wodurch ein Wärmeträger W2 entsteht;
(g) mindestens ein Teil des Wärmeträgers W2 verdichtet wird, wodurch ein verdichteter Wärmeträger W2.1 entsteht, der einen höheren Druck aufweist als der Wärmeträger W2;
(h) der Wärmeträger W1 mit dem Wärmeträger W2.1 vermischt wird, wodurch ein vermischter Wärmeträger W3 entsteht:
(i) mindestens ein Teil des vermischten Wärmeträgers W3 verdichtet wird, wodurch ein verdichteter Wärmeträger W3.1 entsteht, der einen höheren Druck aufweist als der vermischte Wärmeträger W3;
(j) Energie vom verdichteten Wärmeträger W3.1 auf den Strom im Sumpfverdampfer SV1 übertragen wird.

2. Verfahren nach Anspruch 1, wobei die Ströme BS1a und BS1b zu einem Flashbehälter geführt und dort entspannt werden, wodurch eine flüssige Phase FP1 der Brüdenströme BS1a und BS1b anfällt.

3. Verfahren nach Anspruch 2, wobei die Ströme BS1a und BS1b im Flashbehälter vereint werden und als gemeinsame flüssige Phase FP1 anfallen.

4. Verfahren nach Anspruch 3, wobei zumindest ein Teil FP1a der flüssigen Phase FP1 zur Destillationskolonne DK2 geleitet wird.

5. Verfahren nach Anspruch 4, wobei ein anderer Teil FP1b der flüssigen Phase FP1 als Rücklauf zur Destillationskolonne DK1 zurückgeführt wird.

6. Verfahren nach einem der vorhergehenden Ansprüche, wobei im Sumpf der ersten Destillationskolonne DK1 eine Temperatur im Bereich von 40 bis 110 °C, vorzugsweise 50 bis 100 °C vorliegt.

7. Verfahren nach einem der vorhergehenden Ansprüche, wobei im Sumpf der zweiten Destillationskolonne DK2 eine Temperatur im Bereich von 30 bis 100 °C, vorzugsweise 45 bis 80 °C vorliegt.

8. Verfahren nach einem der vorhergehenden Ansprüche, wobei die Destillationskolonne DK1 zwischen 150 und 300 Böden aufweist.

9. Verfahren nach einem der vorhergehenden Ansprüche, wobei die Destillationskolonne DK2 zwischen 150 und 300 Böden aufweist.

10. Verfahren nach einem der vorhergehenden Ansprüche, wobei der Strom BS2 in einen Flashbehälter entspannt wird, wo eine flüssige Phase FP2 anfällt.

11. Verfahren nach Anspruch 10, wobei zumindest ein Teil FP2a der flüssigen Phase FP2 als Produktstrom aus dem Verfahren ausgeschleust wird und ein anderer Teil FP2b der flüssigen Phase FP2 als Rücklauf zur Destillationskolonne DK2 zurückgeführt wird.

12. Verfahren nach einem der vorhergehenden Ansprüche, wobei der Produktstrom mindestens 99 Gew.-% 1-Buten, vorzugsweise mindestens 99,5 Gew.-% 1-Buten, besonders bevorzugt mindestens 99,6 Gew.-% 1-Buten enthält.

13. Verfahren nach einem der vorherigen Ansprüche, wobei im gesamten Verfahren nur ein einziger Wärmeträger eingesetzt wird.

14. Verfahren nach einem der vorherigen Ansprüche, wobei der Wärmeträger W3.1 nach der Vorwärmung zurückgeführt und als Wärmeträger W teilweise zu Schritt c) und teilweise zu Schritt f) geleitet wird.

15. Verfahren nach einem der vorherigen Ansprüche, wobei der Wärmeträger aus der Gruppe, bestehend aus Wasser; Alkoholen; Alkohol-Wasser-Mischungen; Salz-Wasser-Lösungen; Ammoniak; Mineralöle, wie zum Beispiel Dieselöle; Thermalölen, wie zum Beispiel Silikonöle; biologische Ölen, wie zum Beispiel Limonen; und aromatischen oder aliphatischen Kohlenwasserstoffen, wie zum Beispiel Dibenzyltoluol ausgewählt wird, vorzugsweise Wasser, Methanol, Ethanol, Propanol, n-Pentan, n-Butan, n-Hexan, n-Propan oder Ammoniak, besonders bevorzugt Wasser ist.
